# EUROPEAN PATENT SPECIFICATION

(11) **EP 3 398 618 B1**
(45) Date of publication and mention of the grant of the patent: **09.12.2020**
(21) Application number: 18178016.4
(22) Date of filing: 30.10.2014
(51) Int. Cl.: A61L 2/14, A61K 33/00, A61K 33/08, A61M 15/02, A61M 16/10, A61P 3/10, A61P 7/00, A61P 9/00, A61P 9/10, A61P 9/12, A61P 11/00, A61P 9/08, A61P 11/06, A61P 17/00, A61P 17/02, A61P 17/14, C01B 21/20

(54) **METHODS FOR FORMING A DISCRETE STEAM OF MATTER IN A PLASMA STATE**
VERFAHREN ZUR BILDUNG EINES DISKRETEN DAMPFES EINES STOFFES IM PLASMAZUSTAND
PROCÉDÉS POUR FORMER UNE VAPEUR DISCRÈTE D'UNE MATIÈRE DANS UN ÉTAT DE PLASMA

(30) Priority: 31.10.2013 US 201361898390 P
(43) Date of publication of application: 07.11.2018
(62) Divisional of application: 14857991.5
(73) Proprietor: Origin, Inc., Princeton, NJ 08540 (US)
(72) Inventor: Nelson, Howard, Pennington, NJ New Jersey 08534 (US); Dolgopolsky, Alexander, Richboro, PA Pennsylvania 18954 (US); Preston, Michael, Orangeburg, NY New York 10962 (US); Pohl, Michael, Pelham, AL Alabama 35124 (US); Vasilets, Victor, 142432 Chernogolovka (RU)
(74) Representative: Murgitroyd & Company

(56) References cited:
- WO-A1-93/17741
- US-A1- 2004 009 238
- US-A1- 2007 065 473
- US-B1- 6 432 077

## Description

### Field of the Disclosure

The disclosure relates particularly to methods for forming discrete streams of matter in a plasma state, in which the streams have nitric oxide (NO) as part of their content.

### Background of the Disclosure

Nitric Oxide (NO) gas is a short-lived molecule normally found in a gaseous state. NO is a signaling molecule known to have numerous regulatory, protective and therapeutic properties. Augmenting the body's natural generation of NO by either stimulating increased production of endogenous NO or introducing exogenously-produced NO into the body can improve the body's response to damage, pain, and invading organisms. However, it is difficult to deliver NO into living tissue and, in its gaseous state, NO does not penetrate through the dermis. To be clinically useful, NO must be present in the site of action in a sufficient quantity.

Prior methods for delivering NO include the administration of chemical compounds which release NO chemically into the body. Other methods employ NO pathway agonists and NO antagonists. Still other methods employ high pressure NO gas and sprays. Yet another method involves surrounding a body with sealed vacuum containers into which gaseous NO is introduced. Attempts have also been made to force pressurized nitric oxide through tissue and skin. For various reasons, these methods have yielded limited results. For example, gaseous NO is highly reactive, has low diffusion constant and has extremely short life-time in tissue media.

Another method that has failed to achieve clinical success involves the administration of molecular donors, which has been demonstrated to be problematic because the control of the release of the payload cannot be modulated, nor can the penetration/saturation of the donors be reliably modulated.

There are several solutions that target specific clinical outcomes involving NO. Sildenafil citrate (sold under the brand name VIAGRA), for example, interferes with the down regulation of NO in erectile dysfunction syndrome. Etanercept (sold under the brand name ENBRIL), for example, uses an anti-TNF alpha antibody to do what NO would do in inflammatory diseases of the joint. Most solutions involve affecting the NO pathways, due to the difficulty in stimulating production of NO directly at the site of action. Because of the lack of site specificity of these NO pathway pharmacologics, negative side effects can be serious.

US2007/065473A1 and US2004/009238A1 disclose methods and devices for exposing injured mammalian tissues, in a non-abrasive manner, to an effective amount of exogenous gaseous nitric oxide (gNO) in order to promote healing by reducing the size, duration and severity of wounds as well as controlling the infection by reducing number of pathogens at the site and the surrounding area. WO93/17741A1 discloses a system for producing a mixture comprising nitric oxide and air for treatment of medical conditions.

### Summary

In accordance with the present invention there is provided a method for forming a discrete stream of matter in a plasma state, the method comprising:
providing a nitric oxide (NO) production device having:
   a torch body;
   two electrodes disposed in the torch body and insulated from each other;
   a coolant loop disposed in the torch body and surrounding the two electrodes; and
   a cooled channel cooled by the coolant loop;
forming a stream of matter in the NO production device, the forming comprising channelling air between the two electrodes such that an arc discharge is generated and maintained between the two electrodes to form a NO-containing gas flow from the air in the area between the two electrodes under the effect of the arc discharge; and
withdrawing the NO-containing gas flow through the cooled channel;
wherein the stream of matter has, as part of its content, NO in a concentration from 200 parts per million (ppm) to 1000 ppm; and
outputting the stream of matter from an outlet of the device.

### Brief Description of the Drawings

FIG. 1 illustrates a first exemplary device for producing NO according to the disclosure;
FIG. 2 illustrates a second exemplary device for producing NO according to the disclosure;
FIG. 3 illustrates a third exemplary device for producing NO according to the disclosure; and
FIG. 4 is a flow diagram illustrating a first exemplary method in accordance with an embodiment of the present invention.

### Detailed Description

The present invention will now be described more fully hereinafter with reference to the accompanying drawings, in which preferred embodiments of the invention are shown. The invention, however, may be embodied in many different forms and should not be construed as being limited to the embodiments set forth herein. Rather, these embodiments are provided so that this disclosure will be thorough and complete, and will fully convey the scope of the invention to those skilled in the art. In the drawings, like numbers refer to like elements throughout.

In accordance with the present disclosure, a method and apparatus are presented for creating a discrete stream of matter in a plasma state, where the stream has as part of its content NO. The stream may thereafter be administered to an organism to obtain a therapeutic result, but this administering does not form part of the present invention.

NO application at the surface level (i.e., directed at the skin or open wound) is believed to stimulate the body's own production of NO such that therapeutic effects can be obtained at and around the indication site. Alternatively, methods may exploit the fact that NO in the plasma state is of sufficiently high energy and velocity that it can penetrate through and around cellular membranes. In some cases the NO may pass through biofilms and the stratum corneum to produce therapeutic results in the associated tissue.

Matter in a plasma state that contains NO can be created via several methods. Atmosphere contains nitrogen and oxygen, and thus, sufficient energy in the correct geometry can produce nitric oxide from the gaseous mixture. Energy can be added to transition the gaseous N2 and 02 into the plasma state. In one non-limiting, exemplary embodiment, preformed gaseous in N2-O2mixture can be created and passed through a plasma energy arc that transfers sufficient energy to production of NO in a plasma state.

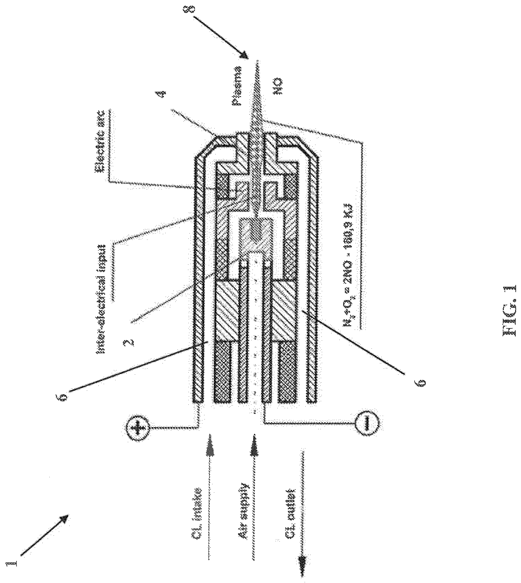

**FIG. 1** shows an exemplary generator portion of a NO production device 1 for use in carrying out one or more of the disclosed methods. As can be seen, air is introduced at a first end of the device 1, and is channeled between a pair of electrodes, namely a cathode 2 and an anode 4 which are insulated from each other. A stationary DC arc discharge is generated and maintained between the electrodes 2, 4. A NO-containing gas flow is formed from the air in the area between the electrodes 2, 4 under the effect of the arc discharge, and is withdrawn through a cooled channel (cooled by a coolant loop 6), enabling NO to be fixed in the flow 8. The temperature of the flow and the NO content can be brought to desired values for providing a therapeutic benefit to a treatment site. Further details regarding the illustrated exemplary NO production device 1 can be found, for example, in U.S. Patent No. 7,498,000 to Pekshev, the entirety of which is incorporated herein by reference.

Classical thermodynamics confirmed by compositional analysis of the thermodynamical equilibrium of air in a plasma state shows that at a temperature lower than 2000°C the concentration of NO in the gas does not exceed 1%. Increasing the plasma temperature increases the NO concentration up to its maximum (∼5%) at a temperature of 3500-4000°C. Slightly less than 4000°C is the temperature of the electrical discharge in the plasma arc of the illustrated device 1. Plasma-chemical reactions, which lead to the formation NO, can be expressed by the following chemical formula:

N₂ + O₂ → 2NO - 180.9 kilo-Joules (kJ)

Life-time of the NO molecule at high temperatures is comparable to the time of its synthesis because of fast reaction of recombination (2NO +O₂ = 2NO₂)+. To prevent recombination and keep therapeutic concentration of NO for medical applications, it is desirable to accomplish rapid cooling of the reaction mixture, *i.e.,* quenching. Quenching of the NO occurs with braking of the escaping flow in surrounding cold air (i.e., from coolant loop 6). The illustrated device 1 enables a direct current plasma arc to be created using ambient air at atmospheric pressure. The device 1 produces a stream of hot air 8 with a composition of plasma species that contains medically significant amount of NO, which in one exemplary embodiment is about 2,500 parts per million (ppm) NO.

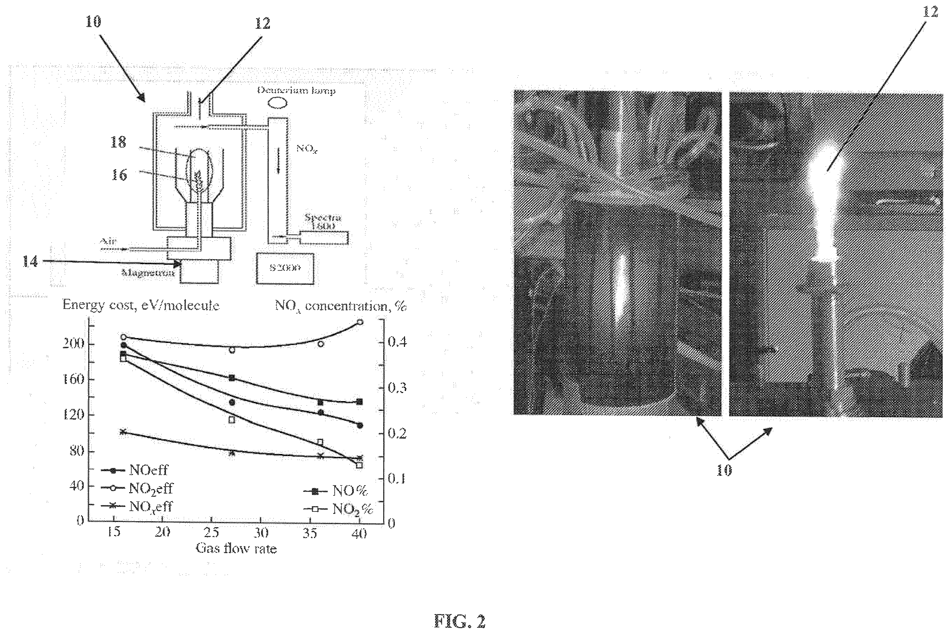

The device 1 shown in **FIG. 1** is not exclusive, and alternative sources of plasma-generated NO may also be used to carrying out one or more of the disclosed methods. **FIG. 2** shows such an alternative device 10 for production of NO-containing matter in a plasma state 12 for use in carrying out one or more of the disclosed methods. This device 10 employs microwave discharge technology for producing matter in a plasma state 12 having a desired composition (i.e., about 2,000 ppm of NO). The illustrated device 10 includes a magnetron 14 having a power of P < 1 kilowatt (kW) and a frequency of 2.45 gigahertz (GHz). Air is passed by the magnetron 14 and directed to a core portion 16 of the torch body 18, where a stream of matter in a plasma state 12 is generated and output for application to a targeted treatment site.

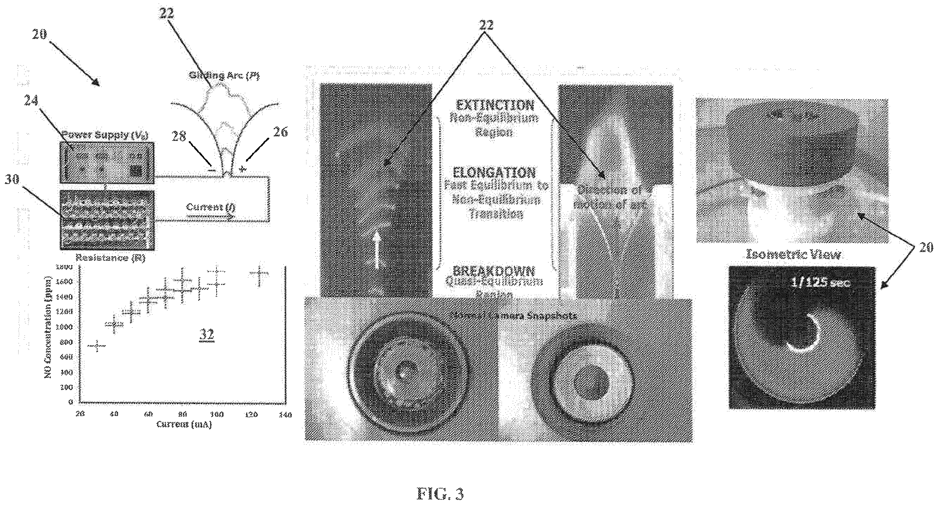

**FIG. 3** shows yet another device 20 for production of NO-containing matter in a plasma state for use in carrying out one or more of the disclosed methods. The illustrated device 20 employs magnetically stabilized gliding arc discharge technology for producing matter in a plasma state 22 having a desired composition (again, about 2,000 ppm of NO).

A gliding arc is operated in air at atmospheric pressure, but at moderate power levels (typically between 50 and 300 Watts). A power source 24 and anode/cathode 26, 28 is employed, and current is restricted using an external ballast resistor 30. This heats the discharge (i.e., the plasma jet 22) to moderate temperatures (2000-3000 degrees Kelvin), while preserving non-equilibrium nature of the discharge (Te>Tg). As such, higher concentrations of NO (e.g., 1600-1800 ppm) can be obtained at lower power input. A graph 32 shows the relationship between NO concentration (ppm) of the matter in a plasma state 22 vs. discharge current (mA).

As will be appreciated, NO in a plasma state can be used for a variety of purposes. For example, NO in the plasma state can be used as an antimicrobial agent. In addition, NO in the plasma state can be used to facilitate hair-growth, as an anti-wrinkle agent, to reduce inflammation, or to facilitate vasodilation. NO in the plasma state further can be employed to alleviate pain associated with osteoarthritis and Rheumatoid Arthritis. It can also be effective in combating Gram Positive microorganisms, Gram Negative microorganisms, Fungi (including onychomycosis) and viruses. It is also therapeutic in treating osteoporosis, collagen formation, stem cell signaling, satellite cell differentiation, wound-healing, wound-management, reduction in scar tissue, remediation of activity related injury, and acne. NO in a plasma state can also aid in nerve regeneration, can inhibit cancer cell proliferation, can promote apoptosis, can stimulate endogenous nitric oxide production, and can stimulate iNOS pathways.

In practice, the NO in a plasma state can be applied directly to or adjacent to living tissue in order to produce the desired effect. It can effectively function to maintain homeostasis in the cardiovascular and respiratory systems. NO, as a signaling molecule, can cause vasodilation which promotes blood vessel flexibility, eases blood pressure, cleans the blood, reverses atherosclerosis and effectively prevents cardiovascular diseases and aids in its recovery. Another important function of NO is slowing down atherosclerotic plaque deposition on vascular walls. NO also plays an active defense role in the immune system. It is a strong antioxidant, and can suppress bacterial infections, viruses and parasitic attacks. It can even deter some types of cancer cell growth. In patients with moderate to severe diabetes, NO can prevent many common and serious complications. NO can also significantly reduce the pain associated with joint swelling in arthritis. NO can effectively decrease the risk of cancer, diabetes, myocardial infarction and stroke.

In the nervous and endocrine systems, NO can induce normal functioning of various body organs. NO can permeate freely through the cell membrane for biological signaling, adjust cellular activities and lead every organ to complete its function properly, including the lungs, liver, kidneys, stomach, heart, brain and genitals. NO can increase blood flow to the genital organs to maintain normal sexual function. The brain transmits signals via its surrounding nerves to the perineal region to provide it with sufficient NO to cause vascular dilation, increasing blood flow to enhance erectile function. Under some conditions, weak erections are the results of insufficient NO production by nerve endings.

NO can also slow the aging process and improve memory. The NO molecules produced by the immune system are not only capable of destroying invading microorganisms, but also help activate and nourish brain cells, significantly slowing aging and improving memory.

### EXEMPLARY INDICATIONS

A non-limiting listing of exemplary indications for which the disclosed NO-containing matter in a plasma state may find beneficial use as a treatment includes:
**Skin infections and wound-healing**
**Skin infections**
   **In chronic wounds**
      Gram-positive
      Gram-negative
   **In acute wounds**
      Gram-positive
      Gram-negative
   **In sub-acute wounds**
      Gram-positive
      Gram-negative
**Fungal infections**
   Tinea pedis
   Onychomycosis
      Toes
      Fingers
**Wound-healing**
   Chronic
      Diabetic foot ulcers
      Decubitus ulcers
   Acute
   Sub-acute
   Burns
      First degree
      Second degree
      Third degree
**Pain and inflammation**
   **Osteo-arthritis**
      Knee
      Hand
      Ankle
      Shoulder
      Elbow
      Toe
      Spine and neck
      Hip
      Finger
   **Rheumatoid arthritis**
      Knee
      Hand
      Ankle
      Shoulder
      Elbow
      Toe
      Spine and neck
      Hip
      Finger

An exemplary baseline composition of matter in a plasma state is shown in **Table 1** below. This baseline composition of matter can be employed with any of the following Exemplary Treatment Schemes, which will be described in more detail later. It will be appreciated, however, that this composition itemization is merely exemplary, and that other compositions can also be used to beneficial effect.

**TABLE 1**

| **BASELINE PLASMA COMPOSITION** | | |
|---|---|---|
| **Parameter** | **Minimum Value** | **Maximum Value** |
| Nitric Oxide (NO) | 200 ppm | 1000 ppm |
| NO₂ | 0 ppm | 25 ppm |
| N₂ | 75 vol% | 78 vol% |
| O₂ | 18 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 ppm | 10000 ppm |

An exemplary baseline treatment scheme is shown in **Table 2** below. This baseline treatment scheme can be employed with any of the following Exemplary Treatment Schemes. It will be appreciated that this baseline scheme may be adjusted, as will be described in relation to a number of Examples to follow, to provide a desired treatment plan for an affected area and in response to a particular indication.

As shown in Table 2, the treatment variables include "distance from exit to site," "time of application," "number of treatments," "length of time between treatments," "temperature of plasma stream at contact with site," and "velocity of plasma stream at contact with treatment site."

"Distance from exit to site" will be understood to be the standoff distance, in centimeters, from the outlet of the plasma device (e.g., device 1, 10, 20) to the treatment site. "Time of application" will be understood to be the amount of time, in seconds, that the NO-containing matter in a plasma state will be directed from the plasma device onto the treatment site, per square centimeter of site area. Thus, the time of application will depend upon the size of the area being treated. "Number of treatments" will be understood to be the discrete number of treatments to be applied at the site. "Length of time between treatments" will be understood to be the amount of time elapsed between applications of the NO-containing matter in a plasma state at the treatment site. "Temperature of plasma stream at contact with treatment site" will be understood to be the temperature of the NO-containing matter in a plasma state, in degrees Celsius, at the treatment site. "Velocity of plasma stream at contact with treatment site" will be understood to be the speed of the NO-containing matter in a plasma state, in meters per second, at the treatment site. Minimum and maximum values are provided for each, recognizing that individual treatment specifications for particular indications will vary within the indicated ranges.

**TABLE 2**

| **BASELINE TREATMENT SCHEME** | | |
|---|---|---|
| **Parameter** | **Minimum Value** | **Maximum Value** |
| Distance from exit to site (cm) | 1 | 25 |
| Time of Application (sec/cm²) | 5 | 45 |
| Number of treatments | 1 | 24 |
| Length of time between treatments (hours) | 3 | 168 |
| Temperature of plasma stream at contact with treatment site °C | 10 | 60 |
| Velocity of Plasma Stream at contact with treatment site (m/sec) | 0.5 | 200 |

A series of Exemplary Treatment Schemes will now be discussed in relation to various indications. Except where indicated otherwise, these treatment schemes assume use of matter in a plasma state having the compositions identified in **Table 1.**

### EXAMPLE 1

### Treatment Scheme - Gram Positive Bacteria

See **Table 1B** below, for partial list of gram positive pathogens. See **Table 1C,** below, for partial list of conditions that present with pathogens from Table **1B.**

The minimum treatment values and maximum treatment values are identified below are based on severity of the gram positive bacterial infection. Severity of the infection is determined by the surface area, depth, colony count and symptoms. Application of therapy increases in intensity, duration and frequency as the severity increases. Minimum treatment parameters define the requirements for the initiation of the decolonization process.

**Example 1 - Table 1A**

| **Parameter** | **Minimum Value** | **Maximum Value** |
|---|---|---|
| Distance from exit to site (cm) | 1 | 25 |
| Time of Application (sec/cm²) | 5 | 45 |
| Number of treatments | 1 | 24 |
| Length of time between treatments (hours) | 3 | 168 |
| Temperature of plasma stream at contact with Site°C | 10 | 60 |
| Velocity of Plasma Stream at contact with Site (m/sec) | 0.5 | 200 |

**Example 1 - Table 1B**

| **Pathogens Susceptible to Plasma/NO therapy** |
|---|
| MRSA |
| MDSA |
| Staphylococcus Aureus |
| Streptococcus A |
| Streptococcus B |
| C. Difficile |
| Streptococcus Mutans |
| Myco Bacterium Tuberculosis |
| Bacillus subtilis |
| Streptococcus Pneumoniae |
| Vancomycin Resistant Enterococcus Faecium |

**Example 1 - Table 1C**

| **Conditions that Present with Pathogens in Table 1B** |
|---|
| Venous Ulcers |
| Pressure Ulcers |
| Decubitus Ulcers |
| Sickle Cell Ulcerations |
| Pyodermas |
| Ulcerated Lesions |
| Vasculitis |
| Diabetic Foot Ulcers |
| Folliculitis |
| Cellulitis |
| Myositis |
| Infections from Animal Bites |
| Surgical Site Infections |
| Catheterizations |
| Carbuncles |
| Furuncles |
| Abscesses |
| Erysipeloid |
| Erysipelas |
| Keratolysis |
| Dermatitis |
| Skin Tuberculosis |
| Impetigo |
| Actinomytosis |
| Leishmaniasis |
| Herpes Simplex |
| Herpetic Neuralgia |
| Skin Flaps |
| Skin Grafts |
| Burns |
| Traumatic Wounds |
| Complicated SSTI |

### EXAMPLE 2

### Treatment Scheme - Gram Negative Bacteria

See **Table 2B** for partial of gram negative pathogens. See **Table 2C** for partial list of conditions that present with pathogens from **Table 2B.**

The minimum treatment values and maximum treatment values are based on severity of the gram negative bacterial infection. Severity of the infection is determined by the surface area, depth, colony count and symptoms. Application of therapy increases in intensity, duration and frequency as the severity increases. Minimum treatment parameters define the requirements for the initiation of the decolonization process. Gram negative bacteria are more difficult to kill than gram positive, so longer treatments are required to decolonize.

**Example 2 - Table 2A**

| **Parameter** | **Minimum Value** | **Maximum Value** |
|---|---|---|
| Distance from exit to site (cm) | 1 | 25 |
| Time of Application (sec/cm²) | 10 | 90 |
| Number of treatments | 1 | 24 |
| Length of time between treatments (hours) | 3 | 168 |
| Temperature of plasma stream at contact with Site°C | 10 | 60 |
| Velocity of Plasma Stream at contact with Site (m/sec) | 0.5 | 200 |

**Example 2 - Table 2B**

| **Gram Negative Pathogens** |
|---|
| Escherichia coli |
| Salmonella |
| Klebsiella pneumoniae |
| Serratia marcescens |
| Enterobacter aerogenes |
| Stenotrophomonas maltopilia |
| Pseudomonas aeruginosa |
| Acinetobacter baumannii |
| Proteus Vulgaris |
| Pantoea agglomerans |

**Example 2 - Table 2C**

| **Conditions That Present with Pathogens in Table 2B** |
|---|
| Venous Ulcers |
| Pressure Ulcers |
| Decubitus Ulcers |
| Sickle Cell Ulcerations |
| Pyodermas |
| Ulcerated Lesions |
| Vasculitis |
| Diabetic Foot Ulcers |
| Folliculitis |
| Cellulitis |
| Myocitis |
| Infections from Animal Bites |
| Surgical Site Infections |
| Catheterizations |
| Carbuncles |
| Furuncles |
| Abscesses |
| Erysipeloid |
| Erysipelas |
| Keratolysis |
| Dermatitis |
| Skin Tuberculosis |
| Impetigo |
| Actinomytosis |
| Leishmaniasis |
| Herpes Simplex |
| Herpetic Neuralgia |
| Skin Flaps |
| Skin Grafts |
| Burns |
| Traumatic Wounds |
| Complicated SSTI |

### EXAMPLE 3

### Treatment Scheme - Wounds - Pressure Ulcers

Severity Classification subject to **Table 3B.** Clinical Presentation subject to **Table 3C,**

The minimum treatment values and maximum treatment values are based on severity of pressure ulcer wound. Severity of the infection is determined by the surface area, depth, and symptoms. Application of therapy increases in intensity, duration and frequency as the severity increases. Minimum treatment parameters define the requirements for the initiation of the wound care management process.

**Example 3 - Table 3A**

| **Parameter** | **Minimum Value** | **Maximum Value** |
|---|---|---|
| Distance from exit to site (cm) | 1 | 25 |
| Time of Application (sec/cm²) | 1 | 45 |
| Number of treatments | 1 | 200 |
| Length of time between treatments (hours) | 3 | 168 |
| Temperature of plasma stream at contact with Site °C | 10 | 60 |
| Velocity of Plasma Stream at contact with Site (m/sec) | 0.5 | 200 |

**Example 3 - Table 3B**

| **Deep Tissue Wounds (Pressure Ulcer Classification)** |
|---|
| Stage I - Intact skin with non blanchable redness usually over a bone |
| Stage II - Partial thickness loss of dermis with open ulcer |
| Stage III - Full thikness tissue loss sub-cutaneous fat may be exposed but not bone, tendon or muscle |
| Stage IV - Full thickness tissue loss with exposed bone, tendon or muscle |
| Unstageable - Full thickness tissue loss in which the base of the ulcer is covered by slough, until it is removed to expose the base of the wound the true depth and therefore stage cannot be determined. |

**Example 3 - Table C**

| **Other Classifications of Wounds** |
|---|
| Surgical |
| Traumatic |
| Chronic |
| Acute |
| Sub-dermal |
| Dermal |

### EXAMPLE 4

### Treatment Scheme - Wounds - Neuropathic Ulcers

Severity Classification is subject to **Table 4B.** Clinical Presentation is subject to **Table 4C.**

The minimum treatment values and maximum treatment values are based on severity of neuropathic ulcer wound. Severity of the wound is determined by the surface area, depth, and symptoms. Application of therapy increases in intensity, duration and frequency as the severity increases. Minimum treatment parameters define the requirements for the initiation of the wound care management process.

**Example 4 - Table 4A**

| **Parameter** | **Minimum Value** | **Maximum Value** |
|---|---|---|
| Distance from exit to site (cm) | 1 | 25 |
| Time of Application (sec/cm²) | 5 | 90 |
| Number of treatments | 1 | 200 |
| Length of time between treatments (hours) | 3 | 168 |
| Temperature of plasma stream at contact with Site °C | 10 | 60 |
| Velocity of Plasma Stream at contact with Site (m/sec) | 0.5 | 200 |

**Example 4 - Table 4B**

| **Severity Classification** |
|---|
| Grade 1 - Superficial ulcer |
| Grade 2 - Penetration into tendon or joint capsule |
| Grade 3 - Involvement of deeper tissues |
| Grade 4 - Gangrene of the forefoot |
| Grade 5 - Gangrene involving more than two-thirds of the foot |

**Example 4 - Table 4C**

| **Other Classifications of Wounds** |
|---|
| Surgical |
| Traumatic |
| Chronic |
| Acute |
| Sub-dermal |
| Dermal |

### EXAMPLE 5

### Treatment Scheme - Wounds - Venous Ulcers

Severity Classification is subject to **Table 5B.** Clinical Presentation is subject to **Table 5C.**

The minimum treatment values and maximum treatment values are based on severity of pressure venous wound. Severity of the wound is determined by the surface area, depth, and symptoms. Application of therapy increases in intensity, duration and frequency as the severity increases. Minimum treatment parameters define the requirements for the initiation of the wound care management process. Treatment includes a border around the wound site of up to 4 cm due to circulatory issues.

**Example 5 - Table 5A**

| **Parameter** | **Minimum Value** | **Maximum Value** |
|---|---|---|
| Distance from exit to site (cm) | 1 | 25 |
| Time of Application (sec/cm²) | 5 | 45 |
| Number of treatments | 1 | 24 |
| Length of time between treatments (hours) | 3 | 168 |
| Temperature of plasma stream at contact with Site °C | 10 | 60 |
| Velocity of Plasma Stream at contact with Site (m/sec) | 0.5 | 200 |

**Example 5 - Table 5B**

| **Severity Classification** |
|---|
| Grade 1 - Superficial ulcer |
| Grade 2 - Penetration into tendon or joint capsule |
| Grade 3 - Involvement of deeper tissues |
| Grade 4 - Gangrene of the forefoot |
| Grade 5 - Gangrene involving more than two-thirds of the foot |

**Example 5 - Table 5C**

| **Other Classifications of Wounds** |
|---|
| Surgical |
| Traumatic |
| Chronic |
| Acute |
| Sub-dermal |
| Dermal |

### EXAMPLE 6

### Treatment Scheme - Wounds - Burns

Severity Classification is subject to **Table 6B.**

The minimum treatment values and maximum treatment values are based on severity of the burn. Severity of the burn is determined by the surface area, depth, and symptoms. Application of therapy increases in intensity, duration and frequency as the severity increases. Distance from the burn site dependent on patient's pain threshold. Minimum treatment parameters define the requirements for the initiation of the burn care management process.

**Example 6 - Table 6A**

| **Parameter** | **Minimum Value** | **Maximum Value** |
|---|---|---|
| Distance from exit to site (cm) | 10 | 30 |
| Time of Application (sec/cm²) | 10 | 90 |
| Number of treatments | 1 | 200 |
| Length of time between treatments (hours) | 1 | 168 |
| Temperature of plasma stream at contact with Site °C | 10 | 50 |
| Velocity of Plasma Stream at contact with Site (m/sec) | 0.5 | 200 |

**Example 6 - Table 6B**

| **Severity Classification** |
|---|
| Stage I Superficial |
| Stage II Superficial partial thickness skin loss |
| Stage III Deep partial thickness skin loss |
| Stage IV Full thickness dermal |
| Stage V Subdermal extending into muscle |

### EXAMPLE 7

### Treatment Scheme - Osteoarthritis - Small Joints

See **Table 7B** for list of locations on body where the Small Joint treatment protocol applies.

The minimum treatment values and maximum treatment values are based on severity of inflammation, mobility and pain. Severity of the arthritis is determined by the level of inflammation, mobility and pain symptoms. Application of therapy increases in intensity, duration and frequency as the severity increases. Minimum treatment parameters define the requirements for the initiation of the osteoarthritis care management process. Treatment includes a border around the wound site of up to 1 cm due to circulatory issues.

**Example 7 - Table 7A**

| **Parameter** | **Minimum Value** | **Maximum Value** |
|---|---|---|
| Distance from exit to site (cm) | 5 | 25 |
| Time of Application (sec/cm²) | 10 | 45 |
| Number of treatments | 3 | 40 |
| Length of time between treatments (hours) | 12 | 168 |
| Temperature of plasma stream at contact with Site°C | 10 | 60 |
| Velocity of Plasma Stream at contact with Site (m/sec) | 0.5 | 200 |

**Example 7 - Table 7B**

| **Body Locations** |
|---|
| Fingers |
| Toes |
| Hand |
| Wrist |
| Elbows |
| Ankles |

### EXAMPLE 8

### Treatment Scheme - Osteoarthritis - Large Joints

See **Table 8B** for list of locations on body where the Large Joint treatment protocol applies.

The minimum treatment values and maximum treatment values are based on severity of inflammation, mobility and pain. Length of time is different from small joint due to the depth of the joint beneath the surface of the skin and the amount of surrounding soft tissue. Severity of the osteoarthritis is determined by the level of inflammation, mobility and pain symptoms. Application of therapy increases in intensity, duration and frequency as the severity increases. Minimum treatment parameters define the requirements for the initiation of the osteoarthritis care management process. Treatment includes a border around the wound site of up to 1 cm due to circulatory issues.

**Example 8 - Table 8A**

| **Parameter** | **Minimum Value** | **Maximum Value** |
|---|---|---|
| Distance from exit to site (cm) | 1 | 25 |
| Time of Application (sec/cm²) | 15 | 120 |
| Number of treatments | 3 | 50 |
| Length of time between treatments (hours) | 12 | 168 |
| Temperature of plasma stream at contact with Site°C | 10 | 60 |
| Velocity of Plasma Stream at contact with Site (m/sec) | 0.5 | 200 |

**Example 8 - Table 8B**

| **Body Locations** |
|---|
| Knee |
| Hip |
| Shoulder |
| Spine |
| Neck |

### EXAMPLE 9

### Treatment Scheme - Rheumatoid Arthritis - Small Joints

See **Table 9B** for list of locations on body where the Small Joint treatment protocol applies.

The minimum treatment values and maximum treatment values are based on severity of inflammation, mobility and pain. Severity of the rheumatoid arthritis is determined by the level of inflammation, mobility and pain symptoms. Application of therapy increases in intensity, duration and frequency as the severity increases. Minimum treatment parameters define the requirements for the initiation of the rheumatoid arthritis care management process. Treatment includes a border around the wound site of up to 3 cm due to circulatory issues.

**Example 9 - Table 9A**

| **Parameter** | **Minimum Value** | **Maximum Value** |
|---|---|---|
| Distance from exit to site (cm) | 3 | 25 |
| Time of Application (sec/cm²) | 30 | 90 |
| Number of treatments | 3 | 40 |
| Length of time between treatments (hours) | 12 | 168 |
| Temperature of plasma stream at contact with Site °C | 10 | 60 |
| Velocity of Plasma Stream at contact with Site | 0.5 | 200 |
| (m/sec) | | |

**Example 9 - Table 9B**

| **Body Locations** |
|---|
| Fingers |
| Toes |
| Hand |
| Wrist |
| Elbows |
| Ankles |

### EXAMPLE 10

### Treatment Scheme - Rheumatoid Arthritis - Large Joints

See **Table 10B** for list of locations on body where the Large Joint treatment protocol applies

The minimum treatment values and maximum treatment values are based on severity of inflammation, mobility and pain. Length of time is different from small joint due to the depth of the joint beneath the surface of the skin and the amount of surrounding soft tissue. Severity of the rheumatoid arthritis is determined by the level of inflammation, mobility and pain symptoms. Application of therapy increases in intensity, duration and frequency as the severity increases. Minimum treatment parameters define the requirements for the initiation of the rheumatoid arthritis care management process. Treatment includes a border around the wound site of up to 1 cm due to circulatory issues.

**Example 10 - Table 10A**

| **Parameter** | **Minimum Value** | **Maximum Value** |
|---|---|---|
| Distance from exit to site (cm) | 1 | 25 |
| Time of Application (sec/cm²) | 20 | 120 |
| Number of treatments | 3 | 60 |
| Length of time between treatments (hours) | 12 | 168 |
| Temperature of plasma stream at contact with Site °C | 10 | 60 |
| Velocity of Plasma Stream at contact with Site (m/sec) | 0.5 | 200 |

**Example 10 - Table 10B**

| **Body Locations** |
|---|
| Knee |
| Hip |
| Shoulder |
| Spine |
| Neck |

### EXAMPLE 11

### Treatment Scheme - Hair Follicle Stimulation

An exemplary composition of matter in a plasma state for use in hair follicle stimulation is shown in **Table 11A** below. It will be appreciated that this composition itemization is merely exemplary, and that other compositions can also be used to beneficial effect. Minimum treatment values and maximum treatment values in **Tables 11A** and **11B** are based on skin type and age. Minimum treatment parameters define the requirements for initiation of the follicle stimulation process.

**Example 11 - Table 11A**

| **PLASMA COMPOSITION** | | |
|---|---|---|
| **Parameter** | **Minimum Value** | **Maximum Value** |
| Nitric Oxide (NO) | 200 ppm | 800 ppm |
| NO₂ | 0 ppm | 25 ppm |
| N₂ | 75 vol% | 95 vol% |
| O₂ | 3 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 m | 10000 ppm |

**Example 11 - Table 11B**

| **Parameter** | **Minimum Value** | **Maximum Value** |
|---|---|---|
| Distance from exit to site (cm) | 6 | 25 |
| Time of Application (sec/cm²) | 10 | 30 |
| Number of treatments | 10 | 100 |
| Length of time between treatments (hours) | 12 | 72 |
| Temperature of plasma stream at contact with Site°C | 10 | 60 |
| Velocity of Plasma Stream at contact with Site (m/sec) | 0.5 | 200 |

### EXAMPLE 12

### Treatment Scheme - Post Insult Keloidosis

The disclosed use of NO in this Example is for treating keloidosis (scar tissue) that has occurred after an insult for the reduction of keloided mass in the insult site. An exemplary composition of matter in a plasma state for use in treating post insult keloidosis is shown in **Table 12A** below. It will be appreciated that this composition itemization is merely exemplary, and that other compositions can also be used to beneficial effect. Minimum treatment values and maximum treatment values in **Tables 12A** and **12B** are based on depth and breadth of the keloided tissue. Application of therapy increases in intensity, duration and frequency as the severity increases. Minimum treatment parameters define the requirements for the initiation of the conversion of keloided tissue process.

**Example 12 - Table 12A**

| **PLASMA COMPOSITION** | | |
|---|---|---|
| **Parameter** | **Minimum Value** | **Maximum Value** |
| Nitric Oxide (NO) | 200 ppm | 800 ppm |
| NO₂ | 0 ppm | 25 ppm |
| N₂ | 75 vol% | 95 vol% |
| O₂ | 3 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 0 ppm | 500 ppm |
| H2 | 0 ppm | 10000 ppm |

**Example 12 - Table 12B**

| **Parameter** | **Minimum Value** | **Maximum Value** |
|---|---|---|
| Distance from exit to site (cm) | 6 | 25 |
| Time of Application (sec/cm²) | 5 | 30 |
| Number of treatments | 4 | 24 |
| Length of time between treatments (hours) | 12 | 168 |
| Temperature of plasma stream at contact with Site °C | 10 | 60 |
| Velocity of Plasma Stream at contact with Site (m/sec) | 0.5 | 200 |

### EXAMPLE 13

### Treatment Scheme - Pre Insult Keloidosis

This disclosed use of NO in this Example is for reducing treating keloidosis (scar tissue) that is anticipated to occur before an insult (such as a surgical incision) for the reduction of keloided mass in the subsequent insult site. An exemplary composition of matter in a plasma state for use in treating pre insult keloidosis is shown in **Table 13A** below. It will be appreciated that this composition itemization is merely exemplary, and that other compositions can also be used to beneficial effect. Minimum treatment values and maximum treatment values in **Tables 13A** and **13B** are based on depth and breadth of the keloided tissue. Application of therapy increases in intensity, duration and frequency as the severity increases. Minimum treatment parameters define the requirements for the initiation of the conversion of keloided tissue process.

**Example 13 - Table 13A**

| | | |
|---|---|---|
| **PLASMA COMPOSITION** | | |

| **Parameter** | **Minimum Value** | **Maximum Value** |
|---|---|---|
| Nitric Oxide (NO) | 200 ppm | 800 ppm |
| NO₂ | 0 ppm | 25 ppm |
| N₂ | 75 vol% | 95 vol% |
| O₂ | 3 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 ppm | 10000 ppm |

**Example 13 - Table 13B**

| **Parameter** | **Minimum Value** | **Maximum Value** |
|---|---|---|
| Distance from exit to site (cm) | 6 | 25 |
| Time of Application (sec/cm²) | 10 | 30 |
| Number of treatments | 1 | 12 |
| Length of time between treatments (hours) | 3 | 72 |
| Temperature of plasma stream at contact with Site °C | 10 | 60 |
| Velocity of Plasma Stream at contact with Site (m/sec) | 0.5 | 200 |

### EXAMPLE 14

### Treatment Scheme - Asthma

This disclosed use of NO in this Example is for inhalation of NO to treat the signs and symptoms of asthma either as rescue therapy or maintenance therapy against an attack. An exemplary composition of matter in a plasma state for use in treating asthma is shown in **Table 14A** below. It will be appreciated that this composition itemization is merely exemplary, and that other compositions can also be used to beneficial effect. Minimum treatment values and maximum treatment values in **Tables 14A** and **14B** are based on severity of asthmatic episode. Severity of the asthma is determined by the symptoms. Application of therapy increases in intensity, duration and frequency as the severity increases. Minimum treatment parameters define the requirements for the initiation of the asthma management process.

**Example 14 - Table 14A**

| **PLASMA COMPOSITION** | | |
|---|---|---|
| **Parameter** | **Minimum Value** | **Maximum Value** |
| Nitric Oxide (NO) | 50 ppm | 800 ppm |
| NO₂ | 0 ppm | 25 ppm |
| N₂ | 75 vol% 78 vol% | |
| O₂ | 18 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 ppm | 10000 ppm |

**Example 14 - Table 14B**

| **Parameter** | **Minimum Value** | **Maximum Value** |
|---|---|---|
| Distance from exit to site (cm) | 8.5 | 25 |
| Applicant (inhalation) breaths | 2 | 45 |
| Number of treatments | 1 | 42 |
| Length of time between treatments (hours) | 3 | 48 |
| Temperature of plasma stream at contact with Site °C | 10 | 40 |
| Velocity of Plasma Stream at contact with Site (m/sec) | 0.5 | 200 |

### EXAMPLE 15

### Treatment Scheme - Respiratory Infections

This disclosed use of NO in this Example is for inhalation of NO to treat the bacterial lode of gram positive or gram negative infections of the lungs and sinuses and other airways. An exemplary composition of matter in a plasma state for use in treating such infections is shown in **Table 15A** below. It will be appreciated that this composition itemization is merely exemplary, and that other compositions can also be used to beneficial effect. Minimum treatment values and maximum treatment values in **Tables 15A** and **15B** are based on severity of infection. Severity of the infection is determined by the symptoms. Application of therapy increases in intensity, duration and frequency as the severity increases.

Exemplary pathogens are included in **Table 15C,** while exemplary conditions are included in **Table 15D.**

**Example 15 - Table 15A**

| **PLASMA COMPOSITION** | | |
|---|---|---|
| **Parameter** | **Minimum Value** | **Maximum Value** |
| Nitric Oxide (NO) | 50 ppm | 800 ppm |
| NO₂ | 0 ppm | 25 ppm |
| N2 | 75 vol% | 78 vol% |
| O₂ | 18 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 ppm | 10000 ppm |

**Example 15 - Table 15B**

| **Parameter** | **Minimum Value** | **Maximum Value** |
|---|---|---|
| Distance from exit to site (cm) | 8.5 | 35 |
| Application (inhalation) breaths | 2 | 45 |
| Number of treatments | 1 | 42 |
| Length of time between treatments (hours) | 3 | 48 |
| Temperature of plasma stream at contact with Site °C | 10 | 40 |
| Velocity of Plasma Stream at contact with Site (m/sec) | 0.5 | 200 |

**Example 15 - Table 15C**

| **Pathogens** |
|---|
| *Streptococcus pyogenes* a |
| Group A streptococcus in Streptococcal pharyngitis ("Strep Throat") |
| Haemophilus influenzae |
| Streptococcus pneumoniae |
| Corvnebacterium diphtheriae |
| Bordetella pertussis |
| Bacillus anthracis |
| Streptococcus pneumonia |
| chlamydophila pneumoniae |
| mycoplasma pneumoniae' |
| staphylococcus aureus |
| moraxella catarrhalis |
| legionella pneumophila |
| mycobacterium tuberculosis |
| mycobacterium bovis |
| mycobacterium africanum |
| mycobacterium canetti |
| mycobacterium microti |

**Example 15 - Table 15D**

| **Conditions** |
|---|
| Flu |
| common cold |
| laryngitis |
| sinusitis |
| tonsillitis |
| bronchitis |
| pneumonia |
| bronchiolitis |
| tuberculosis |
| rhinitis |
| laryn gotracheitis |
| tracheitis |
| epiglotitis |
| nasopharyngitis |

### EXAMPLE 16

### Treatment Scheme - Viruses

This disclosed use of NO in this Example is for the use of NO to treat viral infections. An exemplary composition of matter in a plasma state for use in treating viruses is shown in **Table 16A** below. It will be appreciated that this composition itemization is merely exemplary, and that other compositions can also be used to beneficial effect. Minimum treatment values and maximum treatment values in **Tables 16A** and **16B** are based on severity of infection. Severity of the infection is determined by the symptoms, surface area, depth, colony count. Application of therapy increases in intensity, duration and frequency as the severity increases. Minimum treatment parameters define the requirements for initiation of the decolonization process.

Exemplary pathogens are included in **Table 16C,** while an exemplary list of conditions that present with the pathogens of **Table 16C** are included in **Table 16D.**

**Example 16 - Table 16A**

| **PLASMA COMPOSITION** | | |
|---|---|---|
| **Parameter** | **Minimum Value** | **Maximum Value** |
| Nitric Oxide (NO) | 200 ppm | 800 ppm |
| NO₂ | 0 ppm | 25 ppm |
| N₂ | 75 vol% | 78 vol% |
| O₂ | 18 vol% | 21 vol% |
| O₃ | 0 ppm | 0.1 ppm |
| H₂O₂ | 0 ppm | 1500 ppm |
| H₂O | 0 ppm | 20000 ppm |
| Ar | 1 ^{∗} 10^4 ppm | 9.1 ^{∗} 10^5 ppm |
| He | 5.2 ppm | 9.1 ^{∗} 10^5 ppm |
| CO | 0 ppm | 50 ppm |
| CO₂ | 300 ppm | 500 ppm |
| H2 | 0 ppm | 10000 ppm |

**Example 16 - Table 16B**

| **Parameter** | **Minimum Value** | **Maximum Value** |
|---|---|---|
| Distance from exit to site (cm) | 6 | 25 |
| Time of Application (sec/cm²) | 5 | 30 |
| Number of treatments | 1 | 20 |
| Length of time between treatments (hours) | 3 | 168 |
| Temperature of plasma stream at contact with Site °C | 10 | 60 |
| Velocity of Plasma Stream at contact with Site (m/sec) | 0.5 | 200 |

**Example 16 - Table 16C**

| **Pathogens** |
|---|
| Herpes Simplex |
| Herpes Zoster |
| herpes gladiatorum |
| viral warts |
| molluskum contagiosum |
| human papilloma virus |
| measles |
| rubella |
| erythema infectiosum |
| pityriasis rosea |
| echovirus |
| adenovirus |
| coxsakievirus |

**Example 16 - Table 16D**

| **Conditions** |
|---|
| Venous Ulcers |
| Pressure Ulcers |
| Decubetus Ulcers |
| Sickle Cell Ulcerations |
| Pyodermas |
| Ulerated Lesions |
| Vasculitis |
| Diabetic Foot Ulcers |
| Folliculitis |
| Cellulitis |
| Myocitis |
| Infections from Animal Bites |
| Sugical Site Infections |
| Catheterizations |
| Carbuncles |
| Furuncles |
| Abcesses |
| Erysipeloid |
| Erysipelas |
| Keratolysis |
| Dermatitis |
| Skin Tuberculosis |
| Impetigo |
| Actinomvtosis |
| Leishmaniasis |
| Hepes Simplex |
| Herpetic Neuralgia |
| Skin Flaps |
| Skin Grafts |
| Burns |
| Traumatic Wounds |
| Complicated SSTI |

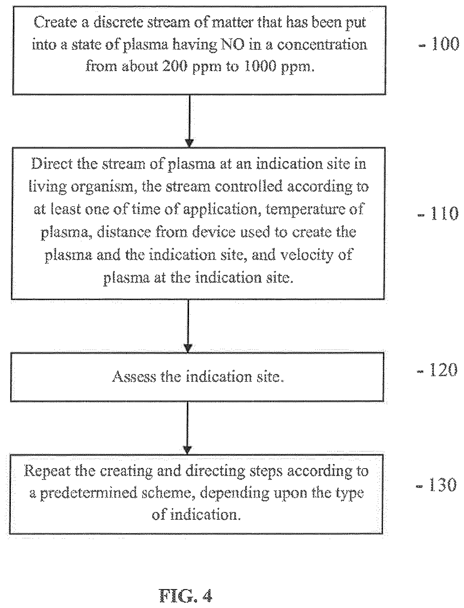

Referring now to **FIG. 4****,** a flow diagram illustrating an exemplary method for administering NO in a plasma state to a treatment site in accordance with the present disclosure is shown. At a first step 100 of the exemplary method, a discrete stream of matter that has been put into a state of plasma may be created, in which the stream has, as part of its content, NO in a concentration from about 200 ppm to 1000 ppm. At step 110, the stream of matter in a plasma state is directed at an indication site in living organism, where the stream is controlled according to at least one of time of application, temperature of the matter in a plasma state, distance from device used to create the matter in a plasma state and the indication site, and velocity of matter in a plasma state at the indication site. At step 120, the indication site is assessed. At step 130, the creating and directing steps are repeated according to a predetermined scheme, depending upon the type of indication.

As used herein, an element or step recited in the singular and proceeded with the word "a" or "an" should be understood as not excluding plural elements or steps, unless such exclusion is explicitly recited. Furthermore, references to "one embodiment" of the present invention are not intended to be interpreted as excluding the existence of additional embodiments that also incorporate the recited features.

## Claims

1. A method for forming a discrete stream of matter in a plasma state, the method comprising:
providing a nitric oxide (NO) production device having: a torch body; two electrodes disposed in the torch body and insulated from each other; a coolant loop disposed in the torch body and surrounding the two electrodes; and a cooled channel cooled by the coolant loop;
forming a stream of matter in the NO production device, the forming comprising:
channeling air between the two electrodes such that an arc discharge is generated and maintained between the two electrodes to form a NO-containing gas flow from the air in the area between the two electrodes under the effect of the arc discharge; and
withdrawing the NO-containing gas flow through the cooled channel;
wherein the stream of matter has, as part of its content, NO in a concentration from 200 parts per million (ppm) to 1000 ppm; and
outputting the stream of matter from an outlet of the device.

2. The method of claim 1, wherein at a distance from 1 to 25 centimeters from the outlet of the device the temperature of the stream of matter is from 10 to 60 degrees C.

3. The method of claim 1, wherein at a distance from 1 to 25 centimeters from the outlet of the device the velocity of the stream of matter is from 0.5 to 200 meters per second.

4. The method of claim 1, wherein the nitric oxide production device is selected from the group comprising an electric arc discharge device, a microwave discharge device and a gliding arc discharge device.

## Patentansprüche

1. Verfahren zum Bilden eines diskreten Stoffstroms in einem Plasmazustand, wobei das Verfahren umfasst:
Bereitstellen einer Stickoxid (NO)-Produktionsvorrichtung mit: einem Brennerkörper; zwei Elektroden, die in dem Brennerkörper angeordnet und voneinander isoliert sind; einem Kühlmittelkreislauf, der in dem Brennerkörper angeordnet ist und die zwei Elektroden umgibt; und einem gekühlten Kanal, der durch den Kühlmittelkreislauf gekühlt wird;
Bilden eines Stoffstroms in der NO-Produktionsvorrichtung, wobei das Bilden umfasst:
Kanalisieren von Luft zwischen den zwei Elektroden, sodass eine Bogenentladung erzeugt und zwischen den zwei Elektroden aufrechterhalten wird, um einen NO-haltigen Gasfluss aus der Luft in dem Bereich zwischen den zwei Elektroden unter der Wirkung der Bogenentladung zu bilden; und
Abziehen des NO-haltigen Gasflusses durch den gekühlten Kanal;
wobei der Stoffstrom als Teil seines Gehalts NO in einer Konzentration von 200 Teilen pro Million (ppm) bis 1000 ppm aufweist; und
Ausgeben des Stoffstroms aus einem Auslass der Vorrichtung.

2. Verfahren nach Anspruch 1, wobei in einem Abstand von 1 bis 25 Zentimeter von dem Auslass der Vorrichtung die Temperatur des Stoffstroms von 10 bis 60 Grad C beträgt.

3. Verfahren nach Anspruch 1, wobei in einem Abstand von 1 bis 25 Zentimeter von dem Auslass der Vorrichtung die Geschwindigkeit des Stoffstroms von 0,5 bis 200 Meter pro Sekunde beträgt.

4. Verfahren nach Anspruch 1, wobei die Stickoxid-Produktionsvorrichtung ausgewählt ist aus der Gruppe, umfassend eine Lichtbogenentladungsvorrichtung, eine Mikrowellenentladungsvorrichtung und eine Gleitbogenentladungsvorrichtung.

## Revendications

1. Procédé pour former un courant discret de matière dans un état de plasma, le procédé comprenant :
la fourniture d'un dispositif de production d'oxyde nitrique (NO) ayant : un corps de torche ; deux électrodes disposées dans le corps de torche et isolées l'une de l'autre ; une boucle d'agent refroidissant disposée dans le corps de torche et entourant les deux électrodes ; et un canal refroidi qui est refroidi par la boucle d'agent refroidissant ;
la formation d'un courant de matière dans le dispositif de production de NO, la formation comprenant :
la canalisation d'air entre les deux électrodes de telle sorte qu'une décharge d'arc est générée et maintenue entre les deux électrodes pour former un écoulement de gaz contenant NO à partir de l'air dans la zone entre les deux électrodes sous l'effet de la décharge d'arc ; et
le retrait de l'écoulement de gaz contenant NO à travers le canal refroidi ;
dans lequel le courant de matière a, en tant que partie de son contenu, du NO en une concentration allant de 200 parties par million (ppm) à 1000 ppm ; et
la sortie du courant de matière d'un orifice de sortie du dispositif.

2. Procédé selon la revendication 1, dans lequel à une distance allant de 1 à 25 centimètres de l'orifice de sortie du dispositif la température du courant de matière va de 10 à 60 degrés C.

3. Procédé selon la revendication 1, dans lequel à une distance allant de 1 à 25 centimètres de l'orifice de sortie du dispositif la vitesse du courant de matière va de 0,5 à 200 mètres par seconde.

4. Procédé selon la revendication 1, dans lequel le dispositif de production d'oxyde nitrique est choisi dans le groupe comprenant un dispositif de décharge à arc électrique, un dispositif de décharge à micro-ondes et un dispositif de décharge à arc glissant.
